# EUROPEAN PATENT SPECIFICATION

(11) **EP 3 583 857 B1**
(45) Date of publication and mention of the grant of the patent: **27.09.2023**
(21) Application number: 19181342.7
(22) Date of filing: 19.06.2019
(51) Int. Cl.: A23L 33/115, A23L 33/00, C11C 3/06

(54) **A PROCESS FOR PRODUCING HUMAN MILK FAT SUBSTITUTES**
VERFAHREN ZUR HERSTELLUNG VON MENSCHLICHEN MUTTERMILCHFETTERSATZSTOFFEN
PROCÉDÉ DE PRODUCTION DE SUBSTITUTS DE MATIÈRES GRASSES DE LAIT HUMAIN

(30) Priority: 20.06.2018 CN 201810637097
(43) Date of publication of application: 25.12.2019
(73) Proprietor: Frutarom Ltd., 2310001 Migdal Haemek (IL)
(72) Inventor: BALTER, Adi, 3438419 Haifa (IL); HOU, Jian Ping, 1839 Qixin Road, Shanghai (CN); ILITZKY-GUR, Maria, 3680301 Nesher (IL); KASTRO, Pnina, 2066832 Yokne'am (IL); BEN-DROR, Gai, 2525700 Gita (IL); HERZOG, Yael, 2281500 Gesher HaZiv (IL)
(74) Representative: J A Kemp LLP

(56) References cited:
- EP-A2- 0 209 327
- EP-B1- 0 209 327
- WO-A1-2008/104381
- WO-A1-2015/177042
- WO-A2-2009/012982
- CN-A- 106 916 631
- CN-A- 107 751 418

## Description

### FIELD OF THE INVENTION

The present invention relates to a field of triglyceride composition production. Particularly, the present invention relates to a process for producing a composition comprising 1,3-dioleoyl-2-palmitoyl glyceride (OPO).

### BACKGROUND OF THE INVENTION

Palmitic acid (C16:0) is the predominant saturated fatty acid in mature human milk, constituting 17% to 25% of the fatty acids. Approximately 70% to 75% of this fatty acid is esterified at the sn-2 position of the triglycerides. In contrast, palmitic acid present in vegetable oils, which are most commonly used in the manufacture of infant formulas, is mainly esterified in the sn-1 and sn-3 positions, whereas the sn-2 position is usually occupied by unsaturated fatty acids.

During triglyceride digestion, the fatty acids esterified in the sn-1 and sn-3 positions are released, yielding two free fatty acids and a 2-monoglyceride. While monoglycerides are well absorbed, the absorption of free fatty acids varies greatly, depending on their chemical structure. Studies have shown that to assure optimal fat absorption, palmitic acid is best absorbed from human milk as sn-2 monoacylglycerol. In contrast, the free palmitic acid originating from the sn-1 and sn-3 positions of vegetable oils, commonly used in manufactured infant formulas, has high tendency to create complexes with dietary minerals such as calcium to form fatty acid soaps, resulting in loss of both calcium and fatty acids in the stool and causing hard stools.

Inspired by the unique structure of triglycerides in human milk and their benefits, some infant formula manufacturers are motivated to produce OPO (1,3-dioleoyl-2-palmitoylglycerol) enriched triglyceride mixtures.

For example, EP0209327 discloses a milk fat substitute comprising 2-palmitic glycerides, in which the sn-1 and sn-3 positions are occupied substantially by different shorter chains and/or unsaturated fatty acids. These glycerides may be obtained by rearrangement of fatty mixtures comprising glycerides consisting substantially of saturated 2-palmityl glycerides, under the influence of an enzyme lipase as rearrangement catalyst which has region-specific activity in the sn-1 and sn-3 positions only of glycerides.

EP0698077 discloses a process for the preparation of triglyceride compositions, in which more than 40wt.% of the total amount of saturated fatty acids are present in the sn-2 position. The compositions may be obtained by a reaction between palm stearin with high oleic sunflower acids in the presence of a catalyst.

WO2008/104381 discloses a process for the production of a composition comprising 1,3-dioleoyl-2-palmitoyl glyceride (OPO). The process comprises interesterification of palm oil stearin fractions to form a randomly interesterified palm oil stearin, subjecting the randomly interesterified palm oil stearin to enzymic transesterification with an oleic acid concentrate using 1,3-position selective enzyme and separating palmitic acid or palmitic non-glyceride esters from the product to form a composition comprising OPO glyceride. This publication mentions that linoleic acid may be present in the oleic acid used. A high amount of oleic acid in the concentrate is recommended.

CN107751418A discloses use of palm stearin as a raw material to increase the content of palmitic acid at the sn-2 position through interesterification with a fatty acid mixture comprising lauric acid: myristic acid: oleic acid: linoleic acid at a mass ratio of 3:3:14:5 as acyl donor. Following acid hydrolysis, 1,3-dioleic acid-2-palmitic acid triglyceride is obtained.

As the desired product in the prior art is OPO, generally, the free fatty acid mixture which is commonly used in the interesterification reaction contains high oleic acid levels.

### BRIEF SUMMARY OF THE INVENTION

The invention aims to improve the process of the prior art. On basis of the prior art, the inventors of the current application have found that surprisingly, using a free fatty acid mixture which contains greater than 20wt.% Linoleic acid (C18:2) results in a more efficient reaction and a better product in comparison with conventional free fatty acid mixtures which contain less than 20wt.% Linoleic acid and greater than 50wt.% oleic acid.

In order to achieve the above purpose, in one aspect, the present invention provides a process for producing a composition comprising 1,3-dioleoyl-2-palmitoyl glyceride (OPO), said process comprises subjecting palm stearin to enzymatic transesterification with a free fatty acid mixture, said free fatty acid mixture contains greater than 20wt.% Linoleic acid (C18:2).

In a preferred embodiment of the present invention, wherein the free fatty acid mixture further contains less than 80wt.% Oleic acid (C18:1).

In a preferred embodiment of the present invention, wherein the free fatty acid mixture further contains less than 5wt.% Palmitic acid (C16:0).

In a preferred embodiment of the present invention, wherein the free fatty acid mixture further contains less than 1wt.% Linolenic acid (C18:3).

In a preferred embodiment of the present invention, wherein the Linoleic acid (C18:2) content in the free fatty acid mixture is greater than 25wt.% and lower than 65wt.%.

In a preferred embodiment of the present invention, wherein the Oleic acid (C18:1) content in the free fatty acid mixture is greater than 30wt.% and lower than 70wt.%. In a preferred embodiment of the present invention, wherein the ratio between the content of Oleic acid (C18:1) in the free fatty acid mixture to Linoleic acid (C18:2) content in the free fatty acid mixture is lower than 5.

In a preferred embodiment of the present invention, wherein the ratio between the content of Oleic acid (C18:1) in the free fatty acid mixture to Linoleic acid (C18:2) content in the free fatty acid mixture is greater than 0.5 and lower than 2.5.

In a preferred embodiment of the present invention, wherein the ratio between the content of Oleic acid (C18:1) in the free fatty acid mixture to Linoleic acid (C18:2) content in the free fatty acid mixture is greater than 0.5 and lower than 2.

In a preferred embodiment of the present invention, wherein the ratio between the content of Oleic acid (C18:1) in the free fatty acid mixture to Linoleic acid (C18:2) content in the free fatty acid mixture is greater than 0.5 and lower than 1.5.

In a preferred embodiment of the present invention, wherein the ratio between the content of free fatty acid mixture to the palm stearin is greater than 1 and lower than 3.

In a preferred embodiment of the present invention, wherein the free fatty acid mixture is derived from sunflower oil.

In another aspect, the present invention provides a composition comprising 1,3-dioleoyl-2-palmitoyl glyceride, the composition is prepared by the process as described above.

In still another aspect, the present invention provides a baby food, which comprising the composition as described above.

As described above, the inventors of the present disclosure have shown that using the unique free fatty acid mixture of the invention in the transesterification reaction surprisingly results in an improved product. This is contrary to the belief among the skilled in the art which points to the benefit of using a free fatty acid mixture which is rich in oleic acid, but not rich in linoleic acid. Therefore, the present invention really achieves an unexpected technical effect for the skilled in the art.

Other features and advantages of the present invention will be described in detail in the following detailed description.

### DETAILED DESCRIPTION OF THE INVENTION

The specific embodiments of the present invention will be described in detail below. It should be understood that the specific embodiments described herein are only used to illustrate and explain the present invention.

The endpoints and any values of the ranges disclosed herein are not limited to the exact range or value, and these ranges or values should be understood as including values close to these ranges or values. For the numerical ranges, one or more new numerical ranges can be obtained by combining the endpoint values of the ranges, the endpoint values of the individual ranges, and the individual point values with each other. The scope should be considered as specifically disclosed herein.

As used herein, the term "sn-1", "sn-2" and "sn-3" positions are referred to the positions of the carbon atoms of glyceryl, that is, the positions of the first carbon atom, of the second carbon atom, and of the third carbon atom of glyceryl. For example, the sn-2 position occupied by palmitic acid represents that the position of second carbon atom of glyceryl is bond to the palmitic acid.

As used herein, the terms "C16:0" (or "C16"), "C18:1", "C18:2", "C18:3" refer to the number of carbon atom contained in the hydrocarbon and unsaturation thereof. For example, "C16:0" or "C16" represents the hydrocarbon containing 16 carbon atoms and having an unsaturation of 0; and "C18:1" represents the hydrocarbon containing 18 carbon atoms and having an unsaturation of 1.

As used herein, the term "palm stearin" is referred to a palm oil fraction with a higher melting point, such as 44-56°C, which may be obtained in the conventional process of producing edible palm oil, but it is not limited.

In one aspect, the invention provides a process for producing a composition comprising 1,3-dioleoyl-2-palmitoyl glyceride (OPO), said process comprises subjecting palm stearin to enzymatic transesterification with a free fatty acid mixture, said free fatty acid mixture contains greater than 20wt.% Linoleic acid (C18:2).

According to the present invention, the enzymatic transesterification may be performed under the condition generally used for the enzymatic transesterification in the art. In a preferred embodiment, the enzymatic transesterification may be performed under the presence of a surfactant coated immobilized 1,3-lipase (prepared as described in Applicant's WO00/56869). Such lipase has region-specific activity in the sn-1 and sn-3 positions only of glycerides, which can catalyze the rearrangement at sn-1 and sn-3 of triglyceride.

According to the present invention, the content of Linoleic acid (C18:2) in said free fatty acid mixture is not particularly limited as long as it satisfies the content requirement of the present invention (i.e. more than 20wt.%). In some embodiments according to the invention, the Linoleic acid (C18:2) content in said free fatty acid mixture may be greater than 25wt.%, may be greater than 30wt.%, may be greater than 33wt.%, and may be greaterthan 35wt.%. In some other embodiments according to the invention, the Linoleic acid content in said free fatty acid mixture may be greater than 20wt.% and lower than 60wt.%, may be greater than 30wt.% and lower than 55wt.%, may be greater than 30wt.% and lower than 50wt.%, may be greater than 35wt.% and lower than 48wt.%, may be greater than 25wt.% and lower than 65wt.%, may be greater than 30wt.% and lower than 65wt.%, may be greater than 30wt.% and lower than 60wt.%, may be greater than 35wt.% and lower than 60wt.%, and may be greater than 37wt.% and lower than 47wt.%.

In some embodiments according to the invention, said free fatty acid mixture may further comprises the Oleic acid (C18:1). The Oleic acid (C18:1) content in said free fatty acid mixture may be lower than 80wt.%, may be lower than 70wt.%, may be lower than 65wt.%, may be lower than 60wt.% and may be lower than 57wt.%. In some other embodiments according to the invention, the Oleic acid content in said free fatty acid mixture may be greater than 20wt.% and lower than 80wt.%, may be greater than 30wt.% and lower than 70wt.%, may be greater than 30wt.% and lower than 65wt.%, may be greater than 35wt.% and lower than 65wt.%, and may be greater than 40wt.% and lower than 60wt.%.

In some embodiments according to the invention, said free fatty acid mixture may further comprises the palmitic acid (C16:0). The Palmitic acid (C16:0) content in said free fatty acid mixture may be lower than 5wt.%, may be less than 4wt.%, may be greater than 2wt.% and lower than 5wt.%, may be greater than 2wt.% and lower than 4wt.%, and may be greater than 3wt.% and lower than 4wt.%.

In some embodiments according to the invention, said free fatty acid mixture may further comprises the Linolenic acid (C18:3). The Linolenic acid (C18:3) content in said free fatty acid mixture may be lower than 1wt.%, may be greater than 0.2wt.% and lower than 1wt.%, may be greater than 0.3wt.% and lower than 0.9wt.%, may be greater than 0.4wt.% and lower than 0.9wt.%, may be greater than 0.4wt.% and lower than 0.8wt.%, and may be greater than 0.5wt.% and lower than 0.7wt.%.

According to the present invention, the method of the present invention can be performed with a specific ratio of oleic acid to linoleic acid in the free fatty acid mixture. In some embodiments according to the invention, the ratio between the content of Oleic acid (C18:1) in the free fatty acid mixture to Linoleic acid (C18:2) content in said free fatty acid mixture may be lower than 5, may be lower than 4, may be lower than 3, may be lower than 2, may be lower than 1.8, may be lower than 2.5 and greater than 0.5, and may be lowerthan 2 and greater than 0.5, and may be lower than 1.5 and greater than 1. In a preferred embodiment according to the invention, the ratio between the content of Oleic acid (C18:1) in the free fatty acid mixture to Linoleic acid (C18:2) content in said free fatty acid mixture is lower than 1.5 and greater than 0.5.

In some embodiments according to the invention, said free fatty acid mixture is derived from at least one of the following vegetable oils: sunflower oil, soybean oil, corn oil and canola oil. In a preferred embodiment according to the invention, said free fatty acid mixture is derived from sunflower oil. In a specific embodiment according the invention, said free fatty acid mixture is prepared by mixing various vegetable oils with different free fatty acid composition to reach a desired ratio of each free fatty acid.

According to a specific embodiment of the invention, said free fatty acid mixture contains greater than 20wt.% Linoleic acid (C18:2), less than 80wt.% Oleic acid (C18:1), less than 5wt.% Palmitic acid (C16:0) and less than 1wt.% Linolenic acid (C18:3), wherein the ratio between the content of Oleic acid (C18:1) to Linoleic acid (C18:2) is lower than 5.

According to a specific embodiment of the invention, said free fatty acid mixture contains greater than 30wt.% and less than 60wt.% Linoleic acid (C18:2), greater than 40wt.% and less than 65wt.% Oleic acid (C18:1), greater than 2wt.% and less than 5wt.% Palmitic acid (C16:0), and less than 1wt.% Linolenic acid (C18:3), wherein the ratio between the content of Oleic acid (C18:1) to Linoleic acid (C18:2) is 2 or below.

As a result, the process according to the present invention using a free fatty acid mixture which contains greater than 20wt.% Linoleic acid (C18:2) results in a more efficient reaction and a better product in comparison with conventional free fatty acid mixtures which contain less than 20wt.% Linoleic acid and greater than 80wt.% oleic acid. In some embodiments according to the invention, said composition prepared according to the process of the present invention comprises greater than 15wt.% and lower than 55wt.% palmitic acid moieties out of the total fatty acids (hereinafter also referred as "%total C16:0" or "%total palmitic acid"), may be greater than 15wt.% and lower than 50wt.%, may be greater than 15wt.% and lower than 40wt.%, may be greater than 15wt.% and lower than 38wt.%, may be greater than 15wt.% and lower than 33wt.%, may be greater than 15wt.% and lower than 25wt.%, may be greater than 17wt.% and lower than 24wt.%, may be greater than 17wt.% and lower than 23wt.%, may be greater than 18wt.% and lower than 23wt.%, may be greater than 18wt.% and lower than 22wt.%, may be greater than 19wt.% and lower than 22wt.%, may be greater than 19wt.% and lower than 21wt.%, may be greater than 20wt.% and lower than 50wt.%, may be greater than 25wt.% and lower than 40wt.%, may be greater than 30wt.% and lower than 40wt.%, may be greater than 30wt.% and lower than 38wt.%, may be greater than 30wt.% and lower than 35wt.%, may be greater than 31wt.% and lower than 34wt.% and may be greater than 31wt.% and lower than 33wt.%.

In addition, as described above, it is well-known that palmitic acid is best absorbed as sn-2 monoacylglycerol. In contrast, the free palmitic acid originating from the sn-1 and sn-3 positions of vegetable oils has high tendency to create complexes with dietary minerals such as calcium to form fatty acid soaps, resulting in loss of both calcium and fatty acids in the stool and causing hard stools. Thus, it is advantageous that the level of palmitic acid moieties at the sn-2 position of the glycerol backbones in said composition of total palmitic acid (hereinafter also referred as "%palmitic acid sn-2 ratio" or "%C16:0 at sn-2 ratio") is high enough. In some embodiments according to the invention, the level of palmitic acid moieties at the sn-2 position of the glycerol backbones in said composition may be at least 30wt.% of total palmitic acid, may be at least 38wt.%, may be at least 40wt.%, may be at least 43wt.%, may be at least 44wt.% and may be at least 50wt.%. In some other embodiments according to the invention, the level of palmitic acid moieties at the sn-2 position of the glycerol backbones in said composition may be greater than 30wt.% and lower than 70wt.% of total palmitic acid, may be greater than 40wt.% and lower than 70wt.%, may be greater than 40wt.% and lower than 60wt.% may be greater than 43wt.% and lower than 60wt.%, may be greater than 44wt.% and lower than 55wt.%, may be greater than 50wt.% and lower than 65wt.%, may be greater than 55wt.% and lower than 70wt.%, may be greater than 40wt.% and lower than 55wt.%, and may be greater than 40wt.% and lower than 50wt.% of total palmitic acid. According to the present invention, "%C16:0 at sn-2 ratio" may be calculated by a formula of [(%C16:0 at sn-2)/3]/(%total C16:0)x100, wherein the term "%C16:0 at sn-2" is referred to % palmitic acid at sn-2 out of total sn-2 positioned fatty acids, and the term "%total C16:0" is same as describe above.

In respect of the composition of the product prepared by the process of the present invention, it may include a triglyceride which contains 52 carbons (hereinafter also referred as C52 triglyceride, which is the desired product of the reaction, e.g. OPO), a triglyceride which contains 48 carbons (hereinafter also referred as C48 triglyceride, which is the raw material for the reaction), and a triglyceride which contains 54 carbons (hereinafter also referred as C54 triglyceride, which is the undesired product). Thus, it is advantageous to obtain a product with a composition of higher levels of C52 triglycerides and lower levels of C48 triglycerides and C54 triglycerides, which indicate a better enzymatic activity and an improved product.

In some embodiments according to the invention, the content of triglycerides which contain 48 carbons (% C48 triglycerides) in said composition is 10wt.% or below, may be below 9wt.%, may be below 8wt.%, may be below 7wt.%, may be greater than 3wt.% and lower than 10wt.%, may be greater than 4wt.% and lower than 9wt.% and may be greater than 5wt.% and lower than 8wt.%. In a preferred embodiment according to the invention, the content of triglycerides which contain 48 carbons (% C48 triglycerides) in said composition is lower than 8wt.%. In a further preferred embodiment according to the invention, the content of triglycerides which contain 48 carbons (% C48 triglycerides) in said composition is lower than 6.5wt.%.

In some embodiments according to the invention, the content of triglycerides which contain 52 carbons (% C52 triglycerides) in said composition is 40wt.% or above, may be greater than 42wt.%, may be greater than 45wt.%, may be greater than 40wt.% and lower than 70wt.%, may be greater than 40wt.% and lower than 60wt.%, may be greater than 40wt.% and lower than 50wt.% and may be greater than 40wt.% and lower than 45wt.%. In a preferred embodiment according to the invention, the content of triglycerides which contain 52 carbons (% C52 triglycerides) in said composition is higher than 45wt.%.

In some embodiments according to the invention, the content of triglycerides which contain 54 carbons (% C54 triglycerides) in said composition is below 25wt.%, may be below 20wt.%, and may be below 17wt.%. In a preferred embodiment according to the invention, the content of triglycerides which contain 54 carbons (% C54 triglycerides) in said composition is below 18wt.%.

In another aspect, the present invention provides a composition comprising 1,3-dioleoyl-2-palmitoyl glyceride, the composition is prepared by the process as described above.

In still another aspect, the present invention provides a baby food, which comprising the composition as described above.

### EXAMPLES

In the present description, as well as in the examples provided below, reference is made to fat bases and fat blends. It is to be understood that the term "fat base" or "fat concentrate" or "fat base concentrate" is used to denote the enzymatically prepared lipid composition comprising a mixture of vegetable-derived triglycerides with high sn-2 palmitic acid; while the term "fat blend" is used to denote a lipid composition comprising a fat base and a mixture of edible vegetable oil/s.

As shown below, the fat blend is a fat base comprising mainly triglycerides with high total palmitic and high sn-2 palmitic acid mixed with other edible vegetable oils. The edible vegetable oil/s may be natural vegetable oil/s, randomized vegetable oil/s, interesterified vegetable oils, enzymatically interesterified vegetable oils, at least two vegetable oils which were co-randomized, at least two vegetable oils which were co-interesterified. Generally, this fat blend is used as a fat fraction in infant formulas and can be used in other baby foods such as biscuits, bar, etc., food articles and clinical nutrition products.

### Example 1 - Production of OPO fat bases:

A triglyceride mixture (palm stearin) was blended and stirred in a ratio of 1:2 with the free fatty acid (FFA) mixture 1. To the resulting blend a surfactant coated immobilized 1,3-lipase (prepared as described in Applicant's WO00/56869) was added. The triglyceride mixture, FFA and catalyst were stirred at 50°C for 4 hours and separated from the catalyst by decantation/filtration .

### Examples 2-5

The product was prepared according to the process of Example 1, except that FFA mixture 1 was replaced by FFA mixtures 2-6 to form fat bases 2-6, respectively.

### Comparative example 1

The product was prepared according to the process of Example 1, except that FFA mixture 1 was replaced by cooperative FFA mixture 1 to form comparative fat base 1. In respect of the FFA mixtures 1-6, they are prepared as below:
FFA mixture 1- 275 g of Oleic acid (OA)-enriched FFA were mixed with 275 g of Linoleic acid (LA)-enriched FFA, to yield OA/LA ratio of 1.5;
FFA mixture 2 - 220 g of OA-enriched FFA were mixed with 330 g of LA-enriched FFA, to yield OA/LA ratio of 1.2;
FFA mixture 3 - 170.5 g of OA-enriched FFA were mixed with 379.5 g of LA- enriched FFA, to yield OA/LA ratio of 1;
FFA mixture 4 - 75 g of OA-enriched FFA were mixed with 425 g of LA-enriched FFA, to yield OA/LA ratio of 0.5;
FFA mixture 5 - 310 g of OA-enriched FFA were mixed with 190 g of LA-enriched FFA, to yield OA/LA ratio of 2;
FFA mixture 6 - 360 g of OA-enriched FFA were mixed with 140 g of LA-enriched FFA, to yield OA/LA ratio of 2.6;
Comparative FFA mixture 1 - 500 g of Oleic acid enriched FFA,
Detailed composition of each FFA mixture is shown in table 1.

The FFA mixtures and final fat bases were analyzed for fatty acid composition (area percentage which corresponds to weight percentage) using an extension of AOAC Official Method Ce 1i-07.

Samples were hydrolyzed with Sodium Hydroxide solution. Then, fatty acid methyl esters were extracted using 12% boron trichloride in methanol solution in the presence of hexane. The extracts were analyzed by gas chromatography. Fatty acid composition reported as area percentage corresponds to weight percentage.

**Table 1. Fatty acid composition (wt.%) of FFA mixtures**

| | FFA mixture 1 | FFA mixture 2 | FFA mixture 3 | FFA mixture 4 | FFA mixture 5 | FFA mixture 6 | Comparative FFA mixture 1 |
|---|---|---|---|---|---|---|---|
| C12 (Lauric) | 0.04 | 0.04 | 0.05 | 0.07 | 0.03 | 0.02 | 0.00 |
| C14 (Miristic) | 0.08 | 0.08 | 0.09 | 0.10 | 0.07 | 0.07 | 0.06 |
| C16 (Palmitic) | 3.12 | 3.44 | 3.72 | 4.59 | 2.73 | 2.41 | 1.52 |
| C16:1 (Palmitoleic) | 0.09 | 0.11 | 0.13 | 0.18 | 0.07 | 0.05 | 0.00 |
| C17 (Margaric) | 0.02 | 0.02 | 0.01 | 0.00 | 0.03 | 0.03 | 0.05 |
| C18 (Stearic) | 1.75 | 1.75 | 1.74 | 1.72 | 1.76 | 1.77 | 1.79 |
| **C18:1n9 (Oleic)** | **55.60** | **50.23** | **45.40** | **30.91** | **62.04** | **67.41** | **82.44** |
| C18:1n7 (Vaccenic) | 0.74 | 0.76 | 0.78 | 0.83 | 0.72 | 0.70 | 0.65 |
| **C18:2n6 (Linoleic)** | **37.24** | **42.19** | **46.65** | **60.02** | **31.30** | **26.34** | **12.48** |
| C18:3n6 (γ-Linolenic) | 0.52 | 0.58 | 0.64 | 0.83 | 0.43 | 0.37 | 0.18 |
| C20:0 (Arachidic) | 0.21 | 0.20 | 0.19 | 0.17 | 0.22 | 0.23 | 0.25 |
| C20:1n9 (Eicosenoic) | 0.34 | 0.33 | 0.32 | 0.29 | 0.36 | 0.37 | 0.40 |

### Analysis of OPO fat bases

OPO fat bases which were produced according to the process of the invention, using the unique FFA mixture of the invention, demonstrated unexpected advantages over the OPO fat base which was produced according to a conventional process using a conventional FFA mixture. Specifically, as demonstrated in Table 2, fat bases 1-6 demonstrated higher levels of triglycerides which contain 52 carbons (C52, e.g. OPO) which are the desired product of the reaction. In parallel, in fat bases 1-6 the content of triglycerides which contain 48 carbons (C48) was lower in comparison with comparative fat base 1. C48 triglycerides are the raw material for the reaction and thus lower levels of these triglycerides in the reaction product demonstrate better enzymatic activity.

**Table 2. Analysis of triglycerides (wt.%) in fat bases**

| | Fat base 1 | Fat base 2 | Fat base 3 | Fat base 4 | Fat base 5 | Fat base 6 | Comparative fat base 1 |
|---|---|---|---|---|---|---|---|
| % C48 triglycerides | 6.31 | 6.43 | 6.51 | 6.87 | 7.98 | 8.18 | 8.47 |
| % C52 (e.g. OPO) triglycerides | 48.87 | 48.61 | 48.38 | 47.5 | 45.50 | 45.39 | 44.36 |

### Analysis of OPO fat bases following distillation

Fat bases 1-3 and comparative sample 1 were distilled to remove FFA and analyzed as demonstrated in Table 3. Palmitic acid sn-2 ratio was higher in fat bases 1-3 which were produced using FFA according to the invention (FFA mixtures 1-3 in comparison with comparative fat base 1 which was produced using a conventional FFA (comparative FFA mixture 1). Importantly, fat bases 1-3 also demonstrated higher levels of triglycerides which contain 52 carbons (C52, e.g. OPO) which are the desired product of the reaction. In parallel, in fat bases 1-3 the content of triglycerides which contain 48 and 54 carbons (C48 and C54, respectively) was lower in comparison with comparative fat base 1. C48 triglycerides are the raw material for the reaction and thus lower levels of these triglycerides in the reaction product demonstrate better enzymatic activity. In parallel, since C54 triglycerides are not the desired product, lower levels of these triglycerides demonstrate better selectivity of the enzyme. Lastly, fat base 3 oxidative stability was compared to that of comparative fat base 1. The results are shown in Table 4 and demonstrate lower p-Anisidine and Peroxide values for fat base 3 in comparison with comparative fat base 1. These lower values are indicative of greater oxidative stability of fat bases according to the invention in comparison with conventional fat bases. The results are surprising since due the greater unsaturation of Linoleic acid in comparison with Oleic acid it was expected that fat base 3, which contained greater level of unsaturation, will be more susceptible to oxidation than comparative fat base 1.

**Table 3. Analysis of triglycerides (wt.%) and %C16 at Sn2 position in fat bases**

| | Fat base 1 | Fat base 2 | Fat base 3 | Comparative fat base 1 |
|---|---|---|---|---|
| %C16 at Sn-2 ratio | 53.5 | 53.3 | 52.7 | 51.4 |
| % C48 triglycerides | *6.02* | 6.44 | *5.61* | *6.77* |
| % C52 (e.g. OPO) triglycerides | *47.68* | *46.93* | 45.57 | 42.22 |
| % C54 triglycerides | *17.60* | *16.89* | 16.16 | 24.12 |

**Table 4. Analysis of p-Anisidine and Peroxide values in fat bases**

| | p-Anisidine value **[AnV]** | Peroxide value **[meq/Kg]** |
|---|---|---|
| **Comparative fat base 1** | 10 | 4.3 |
| **Fat base 3** | 7 | 1.9 |

## Claims

1. A process for the production of a composition comprising 1,3-dioleoyl-2-palmitoyl glyceride, said process comprises subjecting palm stearin to enzymatic transesterification with free fatty acid mixture, said free fatty acid mixture contains greater than 20wt.% Linoleic acid.

2. The process according to claim 1, wherein the free fatty acid mixture further contains less than 80wt.% Oleic acid.

3. The process according to claim 1, wherein the free fatty acid mixture further contains less than 5wt.% Palmitic acid.

4. The process according to claim 1, wherein the free fatty acid mixture further contains less than 1wt.% Linolenic acid.

5. The process according to claim 1, wherein the Linoleic acid content in the free fatty acid mixture is greater than 25wt.% and lower than 65wt.%.

6. The process according to claim 2, wherein the Oleic acid content in the free fatty acid mixture is greater than 30wt.% and lower than 70wt.%.

7. The process according to claim 6, wherein the ratio between the content of Oleic acid in the free fatty acid mixture to Linoleic acid content in the free fatty acid mixture is lower than 5.

8. The process according to claim 7, wherein the ratio between the content of Oleic acid in the free fatty acid mixture to Linoleic acid content in the free fatty acid mixture is greater than 0.5 and lower than 2.5.

9. The process according to claim 8 wherein the ratio between the content of Oleic acid in the free fatty acid mixture to Linoleic acid content in the free fatty acid mixture is greater than 0.5 and lower than 2.

10. The process according to claim 9 wherein the ratio between the content of Oleic acid in the free fatty acid mixture to Linoleic acid content in the free fatty acid mixture is greater than 0.5 and lower than 1.5.

11. The process according to any one of claims 1-10, wherein the ratio between the content of free fatty acid mixture to the palm stearin is greater than 1 and lower than 3.

12. The process according to any one of claims 1-10, wherein the free fatty acid mixture is derived from sunflower oil.

13. A process of any one of the preceding claims, wherein the process further comprises producing a baby food comprising the composition .

## Patentansprüche

1. Verfahren zur Herstellung einer Zusammensetzung, die 1,3-Dioleoyl-2-palmitoylglycerid umfasst, wobei das Verfahren die enzymatische Umesterung von Palmstearin mit freiem Fettsäuregemisch umfasst, wobei das freie Fettsäuregemisch mehr als 20 Gew.-% Linolsäure enthält.

2. Verfahren nach Anspruch 1, wobei das freie Fettsäuregemisch ferner weniger als 80 Gew.-% Ölsäure enthält.

3. Verfahren nach Anspruch 1, wobei das freie Fettsäuregemisch ferner weniger als 5 Gew.-% Palmitinsäure enthält.

4. Verfahren nach Anspruch 1, wobei das freie Fettsäuregemisch ferner weniger als 1 Gew.-% Linolensäure enthält.

5. Verfahren nach Anspruch 1, wobei der Linolsäuregehalt in dem freien Fettsäuregemisch größer als 25 Gew.-% und kleiner als 65 Gew.-% ist.

6. Verfahren nach Anspruch 2, wobei der Ölsäuregehalt in dem freien Fettsäuregemisch größer als 30 Gew.-% und kleiner als 70 Gew.-% ist.

7. Verfahren nach Anspruch 6, wobei das Verhältnis zwischen dem Ölsäuregehalt in dem freien Fettsäuregemisch und dem Linolsäuregehalt in dem freien Fettsäuregemisch kleiner als 5 ist.

8. Verfahren nach Anspruch 7, wobei das Verhältnis zwischen dem Ölsäuregehalt in dem freien Fettsäuregemisch und dem Linolsäuregehalt in dem freien Fettsäuregemisch größer als 0,5 und kleiner als 2,5 ist.

9. Verfahren nach Anspruch 8, wobei das Verhältnis zwischen dem Ölsäuregehalt in dem freien Fettsäuregemisch und dem Linolsäuregehalt in dem freien Fettsäuregemisch größer als 0,5 und kleiner als 2 ist.

10. Verfahren nach Anspruch 9, wobei das Verhältnis zwischen dem Ölsäuregehalt in dem freien Fettsäuregemisch und dem Linolsäuregehalt in dem freien Fettsäuregemisch größer als 0,5 und kleiner als 1,5 ist.

11. Verfahren nach einem der Ansprüche 1 bis 10, wobei das Verhältnis zwischen dem Gehalt an freiem Fettsäuregemisch und Palmstearin größer als 1 und kleiner als 3 ist.

12. Verfahren nach einem der Ansprüche 1 bis 10, wobei das freie Fettsäuregemisch aus Sonnenblumenöl gewonnen wird.

13. Verfahren nach einem der vorhergehenden Ansprüche, wobei das Verfahren ferner die Herstellung einer Babynahrung umfasst, die die Zusammensetzung umfasst.

## Revendications

1. Procédé de production d'une composition comprenant du glycéride de 1,3-dioléoyl-2-palmitoyle, ledit procédé comprenant la soumission de la stéarine de palme à une transestérification enzymatique avec un mélange d'acides gras libres, ledit mélange d'acides gras libres contenant plus de 20 % en poids d'acide linoléique.

2. Procédé selon la revendication 1, dans lequel le mélange d'acides gras libres contient en outre moins de 80 % en poids d'acide oléique.

3. Procédé selon la revendication 1, dans lequel le mélange d'acides gras libres contient en outre moins de 5 % en poids d'acide palmitique.

4. Procédé selon la revendication 1, dans lequel le mélange d'acides gras libres contient en outre moins de 1 % en poids d'acide linolénique.

5. Procédé selon la revendication 1, dans lequel la teneur en acide linoléique dans le mélange d'acides gras libres est supérieure à 25 % en poids et inférieure à 65 % en poids.

6. Procédé selon la revendication 2, dans lequel la teneur en acide oléique dans le mélange d'acides gras libres est supérieure à 30 % en poids et inférieure à 70 % en poids.

7. Procédé selon la revendication 6, dans lequel le rapport entre la teneur en acide oléique dans le mélange d'acides gras libres et la teneur en acide linoléique dans le mélange d'acides gras libres est inférieur à 5.

8. Procédé selon la revendication 7, dans lequel le rapport entre la teneur en acide oléique dans le mélange d'acides gras libres et la teneur en acide linoléique dans le mélange d'acides gras libres est supérieur à 0,5 et inférieur à 2,5.

9. Procédé selon la revendication 8, dans lequel le rapport entre la teneur en acide oléique dans le mélange d'acides gras libres et la teneur en acide linoléique dans le mélange d'acides gras libres est supérieur à 0,5 et inférieur à 2.

10. Procédé selon la revendication 9, dans lequel le rapport entre la teneur en acide oléique dans le mélange d'acides gras libres et la teneur en acide linoléique dans le mélange d'acides gras libres est supérieur à 0,5 et inférieur à 1,5.

11. Procédé selon l'une quelconque des revendications 1 à 10, dans lequel le rapport entre la teneur en mélange d'acides gras libres et la stéarine de palme est supérieur à 1 et inférieur à 3.

12. Procédé selon l'une quelconque des revendications 1 à 10, dans lequel le mélange d'acides gras libres est dérivé d'huile de tournesol.

13. Procédé selon l'une quelconque des revendications précédentes, dans lequel le procédé comprend en outre la production d'un aliment pour bébé comprenant la composition.
